(19) Europäisches Patentamt
European Patent Office
Office européen des brevets

(11) EP 1 110 946 A2

(12) **EUROPÄISCHE PATENTANMELDUNG**

(43) Veröffentlichungstag:
27.06.2001 Patentblatt 2001/26

(51) Int Cl.7: **C07C 271/20**, C07C 271/28, C07C 275/26, C07C 275/40, C07C 333/06, C08F 8/30, C08G 83/00, C08G 73/04, C14C 11/00, D06M 13/395, C23C 22/00, D21H 19/00, B27K 5/00

(21) Anmeldenummer: 00124605.7

(22) Anmeldetag: 10.11.2000

(84) Benannte Vertragsstaaten:
AT BE CH CY DE DK ES FI FR GB GR IE IT LI LU MC NL PT SE TR
Benannte Erstreckungsstaaten:
AL LT LV MK RO SI

(30) Priorität: 23.12.1999 DE 19962272

(71) Anmelder: **BASF AKTIENGESELLSCHAFT**
**67056 Ludwigshafen (DE)**

(72) Erfinder:
• **Bruchmann, Bernd, Dr.**
**67251 Freinsheim (DE)**
• **Treuling, Ulrich, Dr.**
**64625 Bensheim (DE)**

(54) **Isocyanatgruppen aufweisende Bausteine sowie ihre Verwendung zur Funktionalisierung oder Modifizierung von Verbindungen oder Oberflächen**

(57) Die Erfindung betrifft Verbindungen der allgemeinen Formel 1

$$OCN\text{-}R^1\text{-}NHCOX\text{-}R^2\text{-}(Y)_n \qquad (1),$$

in der X eine kovalente Bindung zu $R^2$ darstellt oder O, S oder $NR^3$ ist, Y ein Wasserstoffatom oder eine freie funktionelle Gruppe bedeutet und n für eine ganze Zahl von 1 bis 20 steht. Ferner betrifft die Erfindung ein Verfahren zu ihrer Herstellung sowie ihre Verwendung zur Funktionalisierung oder Modifizierung von Verbindungen oder festen Oberflächen, die mindestens eine mit Isocyanat reaktive Gruppe aufweisen.

Printed by Jouve, 75001 PARIS (FR)

**Beschreibung**

Die Erfindung betrifft Verbindungen der allgemeinen Formel 1

$$OCN\text{-}R^1\text{-}NHCOX\text{-}R^2\text{-}(Y)_n \tag{1},$$

in der X eine kovalente Bindung zu $R^2$ darstellt oder O, S oder $NR^3$ ist und Y ein Wasserstoffatom oder eine freie funktionelle Gruppe bedeutet, ein Verfahren zu ihrer Herstellung sowie ihre Verwendung zur Funktionalisierung oder Modifizierung von Verbindungen oder Oberflächen, die mindestens eine mit Isocyanat reaktive Gruppe aufweisen.

Sowohl für herkömmliche funktionalisierte Monomere oder polymere Strukturen, z.B. für monomere oder polymere di-, tri-, oder höherfunktionelle Alkohole oder Amine, als auch für Makromolekül-strukturen mit hohem Verzweigungsgrad und/oder einer großen Anzahl von funktionellen Gruppen an der Molekülperipherie (funktionalisierte Makromoleküle), zu nennen sind hier die Strukturtypen der Dendrimere, der hoch- und hyperverzweigten Polymere und der Sternpolymere, werden zunehmend neue Anwendungsgebiete erschlossen. Für bestimmte Anwendungen ist es notwendig, gegebene funktionelle Gruppen der funktionalisierten Monomere oder Polymere zu maskieren oder aber die funktionellen Gruppen zu variieren. Beispielsweise kann es von Vorteil sein, hydrophile Moleküle zu hydrophobieren oder hydrophobe Moleküle zu hydrophilieren. Weiterhin kann es vorteilhaft sein, z.B. Aminogruppen in Hydroxylgruppen oder Carbonsäuregruppen umzuwandeln oder OH-Gruppen in aktivierbare Doppelbindungen zu transformieren. Weiterhin besteht allgemein Bedarf, funktionelle Gruppen auf Oberflächen zu funktionalisieren oder zu modifizieren, um die Eigenschaften der Oberfläche in geeigneter Weise zu verändern.

WO 97/36857 beschreibt die Verwendung von OH-Gruppen-geschützten Trihydroxyalkylaminoalkanen, die mittels Phosgenierung in ein Monoisocyanat umgewandelt werden. Der resultierende Isocyanat-Baustein wird dazu verwendet, mit Isocyanat reaktive Gruppen in dendrimeren Molekülen zu modifizieren. Jedoch sind bei dieser Methode die Ausgangsprodukte für die Monoisocyanate synthetisch aufwendig herzustellen und das Verfahren ist nur mit kleinen Substanzmengen durchführbar. Ferner können zunächst nur Gruppen in das Makromolekül eingeführt werden, die mit NCO-Gruppen unreaktiv sind. Die Erzeugung von z.B. Hydroxyl- oder Carbonsäuregruppen benötigt den zusätzlichen Reaktionsschritt einer Ether- oder Esterspaltung.

H.W.I. Peerlings und E.W. Meijer, Tetrahedron Lett. 40 (1999) 1021-1024, beschreiben ebenfalls die Verwendung von speziell hergestellten Alkyl- und Aryl-Monoisocyanaten zur Oberflächen-modifikation von Polyamindendrimeren. Auch hier werden nur mit Isocyanaten unreaktive Reste in das Makromolekül eingeführt.

R.M. Versteegen, R.P. Sijbesma und E.W. Meijer, Angew. Chem. 1999, 111, 3095-3097, beschreiben weiterhin die Synthese von [η]-Polyurethanen, bei der durch Phosgenierung von linearen aliphatischen α, ω-Aminoalkoholen in situ Hydroxyisocyanate generiert werden, die in der Reaktionslösung nicht stabil sind und direkt weiter zu linearen Polyurethanen polymerisieren.

Für Bausteine, die es ermöglichen, Verbindungen oder Oberflächen, die mit Isocyanat reaktive Gruppen aufweisen, beliebig zu funktionalisieren oder zu modifizieren, besteht also weiterhin Bedarf.

Der Erfindung lag daher die Aufgabe zugrunde, Isocyanatgruppen aufweisende Bausteine bereitzustellen, die zur Funktionalisierung oder Modifizierung von Verbindungen oder Oberflächen, die mindestens eine mit Isocyanat reaktive Gruppe aufweisen, verwendet werden können. Ferner war es Aufgabe der Erfindung, ein Verfahren zur Herstellung dieser Bausteine bereitzustellen.

Die Aufgaben werden durch Verbindungen der allgemeinen Formel 1

$$OCN\text{-}R^1\text{-}NHCOX\text{-}R^2\text{-}(Y)_n \tag{1},$$

gemäß nachfolgendem Text gelöst.

Gegenstand der Erfindung sind Verbindungen der allgemeinen Formel 1, nachfolgend in dieser Anmeldung als Bausteine bezeichnet,

$$OCN\text{-}R^1\text{-}NHCOX\text{-}R^2\text{-}(Y)_n \tag{1},$$

in der $R^1$ und $R^2$ ein substituierter oder unsubstituierter, linearer oder verzweigter, gesättigter oder ungesättigter Alkylenrest mit 1 bis 20 C-Atomen, bevorzugt 2 bis 20 C-Atomen, mehr bevorzugt 4 bis 20 C-Atomen und besonders bevorzugt 6 bis 20 C-Atomen, ein substituierter oder unsubstituierter, gesättigter oder ungesättigter Cycloalkylenrest mit 3 bis 20 C-Atomen, ein substituierter oder unsubstituierter Arylenrest mit 3 bis 20 C-Atomen, ein Arylenalkylenrest mit 4 bis 20 C-Atomen, ein heterocyclischer Rest oder eine beliebige lineare oder verzweigte Abfolge von zwei oder mehr der genannten Reste, gegebenenfalls verknüpft über Ether-, Thioether-, Ester-, Amin- oder Amid-Strukturen, ist, X eine kovalente Bindung zu $R^2$ ist oder O, S oder $NR^3$ bedeutet, wobei $R^3$ ein Wasserstoffatom oder ein substituierter oder unsubstituierter, linearer oder verzweigter, gesättigter oder ungesättigter Alkylrest mit 1 bis 20 C-Atomen, ein substituierter oder unsubstituierter, gesättigter oder ungesättigter Cycloalkylrest mit 3 bis 20 C-Atomen, ein substituierter oder unsubstituierter Arylrest mit 3 bis 20 C-Atomen, ein heterocyclischer Rest oder eine beliebige lineare oder verzweigte Abfolge von zwei oder mehr der genannten Reste ist, Y eine freie funktionelle Gruppe bedeutet und n für eine ganze Zahl von 1 bis 20, bevorzugt 1 bis 5, besonders bevorzugt 1 bis 3, steht. Y kann ferner ein Wasserstoffatom bedeuten.

Ein weiterer Gegenstand der Erfindung ist ein Verfahren zur Funktionalisierung oder Modifizierung von Verbindungen oder Oberflächen, die mindestens eine mit Isocyanat reaktive Gruppe aufweisen, durch Umsetzung einer Verbindung der allgemeinen Formel 1

$$\text{OCN-R}^1\text{-NHCOX-R}^2\text{-(Y)}_n \tag{1},$$

wobei $R^1$, $R^2$, X, Y und n die vorstehend genannte Bedeutung haben, mit mindestens einer mit Isocyanat reaktiven Gruppe einer Verbindung, die mindestens eine mit Isocyanat reaktive Gruppe aufweist, oder mit mindestens einer mit Isocyanat reaktiven Gruppe auf einer Oberfläche, die mindestens eine mit Isocyanat reaktive Gruppe aufweist.

Ein weiterer Gegenstand der Erfindung ist die Verwendung einer Verbindung der allgemeinen Formel 1

$$\text{OCN-R}^1\text{-NHCOX-R}^2\text{-(Y)}_n \tag{1},$$

in der $R^1$, $R^2$, X, Y und n die vorstehend genannte Bedeutung haben, zur Funktionalisierung oder Modifizierung von Verbindungen oder Oberflächen, die mindestens eine mit Isocyanat reaktive Gruppe aufweisen.

Schließlich ist Gegenstand der Erfindung ein Verfahren zur Herstellungen einer Verbindung der allgemeinen Formel 1

$$\text{OCN-R}^1\text{-NHCOX-R}^2\text{-(Y)}_n \tag{1}$$

durch Umsetzung eines Diisocyanats der allgemeinen Formel 2

$$\text{OCN-R}^1\text{-NCO} \tag{2}$$

mit einer Verbindung der allgemeinen Formel 3

$$\text{HX-}R^2\text{-(Y)}_n \tag{3},$$

wobei $R^1$, $R^2$, $R^3$, X, Y und n vorstehend genannte Bedeutung haben und in Formel 3 X außerdem OCO bedeuten kann.

Unter einem substituierten Alkylenrest ist im Rahmen dieser Erfindung ein Alkylenrest zu verstehen, der an mindestens einer Stelle mit einem $C_1$-$C_6$-Alkylrest, einem $C_3$-$C_8$-Arylrest, einem Halogenatom, ausgewählt aus Fluor, Chlor, Brom oder Iod, oder einem Gemisch davon substituiert ist. Gemäß dieser Definition sind auch die Begriffe substituierter Cycloalkylenrest, substituierter Arylenrest, substituierter Alkylrest, substituierter Cycloalkylrest und substituierter Arylrest zu verstehen.

Unter einer freien funktionellen Gruppe ist im Rahmen dieser Erfindung eine reaktionsfähige Stelle zu verstehen, die nicht durch eine Schutzgruppe geschützt ist. Beispiele für die im Rahmen der Erfindung verwendbaren freien funktionellen Gruppen sind eine Hydroxyl-, Thiol-, eine Nitro-, eine gegebenenfalls substituierte Amino-, eine Amido-, eine Sulfonsäure-, eine Sulfensäure-, eine Sulfinsäure-, eine Sulfonamid-, eine Carbonyl-, eine Carbonsäure-, eine Nitril-, eine Isonitril-, eine Cyanat-, eine Isocyanat-, eine Thiocyanat, eine Isothiocyanat, eine Silyl-, eine Silanyl-, eine gegebenenfalls substituierte Phosphin-, eine Phosphorsäure-, eine Phosphorigsäure-, eine Phosphonat-, eine Acryl-, eine Methacryl-, eine Vinyl-, eine Allyl- oder eine Acetylengruppe oder ein Halogenatom.

Bevorzugt bedeutet in den allgemeinen Formeln Y eine Vinyl-, eine Allyl-, eine Sulfonyl-, eine Sulfenyl-, eine Sulfinyl- eine Sulfonamid-, eine Carbonyl-, eine Silyl-, eine Silanyl, eine Hydroxy-, Thiol-, Carbonsäure-, Sulfonsäure-, Acryl-, Methacryl-oder eine gegebenenfalls substituierte Aminogruppe.

Besonders bevorzugt ist Y eine Hydroxy-, Thiol-, Carbonsäure-, Sulfonsäure-, Acryl-, Methacryl- oder eine gegebenenfalls substituierte Aminogruppe.

Unter einer substituierten Aminogruppe ist im Rahmen dieser Erfindung eine Aminogruppe zu verstehen, die ein- oder zweifach mit einem $C_1$-$C_6$-Alkylrest, einem $C_3$-$C_8$-Arylrest, einem Halogenatom, ausgewählt aus Fluor, Chlor, Brom oder Iod, oder einem Gemisch davon substituiert ist.

Unter einer mit Isocyanat reaktiven Gruppe ist im Rahmen dieser Erfindung eine Gruppe zu verstehen, die über mit NCO-Gruppen reaktive Wasserstoffatome verfügt oder die mit NCO-Gruppen eine Additionsverbindung eingehen kann. Beispiele für diese Gruppen sind OH-, SH-, NH-, COOH-Gruppen, Epoxide, Säureanhydrid- oder Carbodiimidgruppen, davon sind OH-, SH-, NH- oder COOH-Gruppen bevorzugt.

In einer bevorzugten Ausführungsform der erfindungsgemäßen Bausteine bedeutet $R^1$ einen 2,4-Toluylen-, 2,6-Toluylen-, 4,4'-Diphenylmethylen-, 2,4'-Diphenylmethylen-, 3-Alkyl-4,4'-Diphenylmethylen, wobei Alkyl für $C_1$ bis $C_{10}$ steht, 1,3- und 1,4-Phenylen-, 1,5-Naphthylen-, Tolidin-, Diphenylen-, Tetramethylen-, Hexamethylen-, Dodecylen-, Lysinalkylenester-, wobei Alkylen für $C_1$ bis $C_{10}$ steht, Isophoronylen-, 2-Methyl-pentamethylen-, 2,2,4- oder 2,4,4-Trimethyl-1,6-hexamethylen-, 1,4-Cyclohexylen, 3-Methylen-1-methyl-1-cyclohexylen-, 2-Butyl-2-ethylpentamethylen-, 4-Methyl-1,3-cyclohexylen-, 4,4'- und 2,4'-Methylenbis(cyclohexyl)-, Xylylen-, Tetramethylxylylen-, 2-Butyl-2-ethylpentamethylenrest oder ein Gemisch davon und $R^2$ bedeutet einen linearen oder verzweigten, gesättigten oder ungesättigten Alkylen- oder Cycloalkylenrest mit 1 bis 20 C-Atomen, einen substituierten oder unsubstituierten Arylenrest mit 3 bis 20 C-Atomen oder eine beliebige lineare oder verzweigte Abfolge von zwei oder mehr der genannten Reste, die gegebenenfalls über Ether-, Thioether-, Amin-, Amid- oder Estergruppen miteinander verknüpft sein können. Weiterhin sind auch Gemische der genannten Reste $R^2$ zulässig.

Zur Herstellung der erfindungsgemäßen Bausteine werden Diisocyanate mit Alkoholen, Thiolen, primären oder sekundären Aminen oder Carbonsäuren zu den entsprechenden Additionsprodukten umgesetzt, wobei die Reaktion mit Carbonsäuren in der Regel unter $CO_2$-Abspaltung verläuft. Besonders bevorzugt ist die Umsetzung von Diisocyanaten mit Alkoholen, Thioalkoholen und Aminen.

Im Allgemeinen erfolgt die Umsetzung bei einer Temperatur von 0 bis 120°C und die Reaktionszeit beträgt üblicherweise von 5 Minuten bis 24 Stunden. Die Reaktion wird bevorzugt unter Schutzgas mit oder ohne Lösungsmittel, gegebenenfalls unter Zusatz von in der Polyurethanchemie üblichen Katalysatoren durchgeführt. Als Lösungsmittel kommen vorzugsweise solche zum Einsatz, die gegenüber Isocyanatgruppen inert sind. Zu nennen sind hier zum Beispiel Benzol, Toluol, Chlorbenzol, Dichlorbenzol, Diethylether, Tetrahydrofuran, Dioxan, Aceton, 2-Butanon, Essigester, Butylacetat, Chloroform, Methylenchlorid, N-Methylpyrrolidon, Dimethylformamid oder Dimethylacetamid.

In einer bevorzugten Ausführungsform werden als Einsatzstoffe Diisocyanate verwendet, deren Isocyanatgruppen eine unterschiedliche Reaktivität gegenüber der mit Isocyanat reaktiven Komponente aufweisen. Dabei werden die erfindungsgemäßen Bausteine durch Umsetzung von äquimolaren Mengen dieses Diisocyanates mit der mit Isocyanat reaktiven Verbindung der allgemeinen Formel 3 erhalten. Durch den Reaktivitätsunterschied der NCO-Gruppen ist die Selektivität in der Regel hoch genug, so daß die gewünschten Monoaddukte in entsprechender Reinheit entstehen.

In einer weiteren Ausführungsform werden Isocyanate mit gleich reaktiven NCO-Gruppen verwendet. Diese werden üblicherweise in einem 2- bis 15fach, bevorzugt 5- bis 10fach, molaren Überschuß mit der NCO-reaktiven Verbindung der allgemeinen Formel 3 umgesetzt und das überschüssige Isocyanat anschließend entfernt.

Als Isocyanate sind prinzipiell alle organischen Diisocyanate zur Herstellung der erfindungsgemäßen Bausteine geeignet, bevorzugt eingesetzt werden 2,4-Toluylendiisocyanat, 2,6-Toluylendiisocyanat, 4,4'-Diphenylmethandiisocyanat, 2,4'-Diphenylmethandiisocyanat, 3-Alkyl-4,4'-Diphenylmethan-diisocyanat, wobei Alkyl für $C_1$ bis $C_{10}$ steht, 1,3- und 1,4-Phenylendiisocyanat, 1,5-Naphthylendiisocyanat, Tolidindiisocyanat, Diphenyldiisocyanat, Tetramethylendiisocyanat, Hexamethylendiisocyanat, Dodecyldiisocyanat, Lysinalkylesterdiisocyanat, wobei Alkyl für $C_1$ bis $C_{10}$ steht, Isophorondiisocyanat, 2-Methyl-pentamethylendiisocyanat, 2,2,4- oder 2,4,4-Trimethyl-1,6-hexamethylen-diisocyanat, 1,4-Diisocyanatocyclohexan, 3-Isocyanatomethyl-1-methyl-1-isocyanatocyclohexan, 2-Butyl-2-ethylpentamethylen-diisocyanat, 2-Isocyanatopropylcyclohexylisocyanat, 4-Methyl-cyclohexan-1,3-diisocyanat, 4,4'- und 2,4'-Methylenbis(cyclohexyl)diisocyanat, 1,3- oder 1,4-Bis (isocyanatomethyl)cyclohexan, Xylylendiisocyanat, und Tetramethylxylylendiisocyanat (TMXDI).

Besonders bevorzugt sind Isocyanate mit NCO-Gruppen unterschiedlicher Reaktivität, z.B. aromatische Diisocyanate, wie 2,4-Toluylendiisocyanat (2,4-TDI), 2,4'-Diphenylmethandiisocyanat (2,4'-MDI), 3-Alkyl-4,4'-Diphenylmethan-diisocyanat, wobei der Alkylrest 1 bis 10 Kohlenstoffatome enthält, oder aliphatische Diisocyanate, wie Isophorondiisocyanat (IPDI), 2-Butyl-2-ethylpentamethylen-diisocyanat, 2-Isocyanatopropyl-cyclohexyl-isocyanat, 3-Isocyanatomethyl-1-methyl-1-isocyanatocyclohexan, Lysinalkylesterdiisocyanat, wobei Alkyl für $C_1$ bis $C_{10}$ steht, 2,4'-Methylenbis(cyclohexyl)isocyanat (2,4'-HMDI) und 4-Methylcyclohexan-1,3-diisocyanat (H-TDI).

Weiterhin sind Isocyanate besonders bevorzugt, deren NCO-Gruppen zunächst gleich reaktiv sind, bei denen sich jedoch durch Erst-addition eines Alkohols oder Amins an einer NCO-Gruppe ein Reaktivitätsabfall bei der zweiten NCO-Gruppe induzieren läßt. Beispiele dafür sind Isocyanate, deren NCO-Gruppen über ein elektronisches System gekoppelt sind, z.B. 1,3- und 1,4-Phenylendiisocyanat, 1,5-Naphthylendiisocyanat (NDI), Diphenyldiisocyanat, Tolidindiisocyanat oder 2,6-Toluylendiisocyanat (2,6-TDI).

Weiterhin sind Isocyanate besonders bevorzugt, deren NCO-Gruppen gleich reaktiv sind, die sich jedoch leicht aus dem Substanzgemisch destillativ entfernen lassen, z.B. Tetramethylendiisocyanat, Hexamethylendiisocyanat, 1,3- und 1,4-Bis(isocyanatomethyl)-cyclohexan, Tetramethylxylylendiisocyanat, Xylylendiisocyanat, 4,4'-Diphenylmethandiisocyanat oder 4,4'-Methylenbis-(cyclohexyl)-isocyanat.

Zur Herstellung der erfindungsgemäßen Bausteine können auch Mischungen der genannten Isocyanate verwendet werden.

Mit dem erfindungsgemäßen Verfahren kann man Strukturen erzeugen und isolieren, die neben den Isocyanat-Gruppen auch mit Isocyanat reaktive Gruppen enthalten. So weist z.B. das Umsetzungsprodukt aus Tetramethylxylylendiisocyanat (TMXDI) und Diisopropanolamin eine NCO-Gruppe und zwei sekundäre OH-Gruppen auf. Es kann als Festsubstanz isoliert werden und ist bei Raumtemperatur über längere Zeit lagerbar (ca. 24 h).

Beispiele für bevorzugte erfindungsgemäße Bausteine sind Additionsprodukte aus Hexamethylendiisocyanat, Isophorondiisocyanat, Tetramethylxylylendiisocyanat, 2,4'- Toluylendiisocyanat, 4,4'-Diphenylmethandiisocyanat, 2,4'-Diphenylmethandiisocyanat, p-Phenylendiisocyanat und Monoalkoholen, wie zum Beispiel Methanol, Ethanol, Propanol, Butanol, Hexanol, Hexenol, Octanol, Decanol, Dodecanol, Octadecanol, Octadecenol, Allylalkohol, Benzylalkohol, veretherten Monoalkoholen, wie Ethylenglykolmonomethylether, Propylenglykolmonomethylether, Polyethylenglykolmonomethylether oder Polypropylenglykolmonomethylether, Thioalkoholen, wie zum Beispiel Mercaptoethanol, Butanthiol oder Dodecanthiol, Monoaminen, wie zum Beispiel Methylamin, Ethylamin, Propylamin, Butylamin, Dibutylamin, Hexylamin, Octylamin, Decylamin, Anilin oder Benzylamin, Aminogruppen-funktionalisierten Polyalkylenoxyden, Monocarbonsäuren, wie zum Beispiel Essigsäure, Propionsäure, Buttersäure, Hexansäure, Octansäure, Decansäure oder Benzoesäure, Dialkanolaminen, wie zum Beispiel Diethanolamin, Dipropanolamin oder Diisopropanolamin, Trialkanolaminen, wie zum Beipiel Tris(hydroxymethyl)aminomethan oder Tris(hydroxyethyl)aminomethan, Hydroxycarbonsäuren, wie zum Beispiel Hydroxyessigsäure, Hydroxypropionsäure oder Hydroxypivalinsäure, Mercaptocarbonsäuren, wie zum Beispiel Mercaptoessigsäure oder Mercaptopropionsäure, Aminocarbonsäuren, wie zum Beispiel Glycin, ß-Alanin oder Aminocapronsäure, Aminosulfonsäuren, wie zum Beispiel Taurin. Sofern die Säuren in Form ihrer Salze verwendet werden, kommen bevorzugt Natrium-, Kalium- oder Ammoniumsalze in Betracht. Weiterhin kommen als Bausteine Phosphor-haltige Verbindungen, wie zum Beispiel (2-Hydroxyalkyl)triphenylphosphonium-Salze, 1-(Diphenylphosphinoyl)-propan-2-ol, Aminoethyl-diphenylphosphin oder Silizium-haltige Verbindungen, wie zum Beispiel Trimethylsilylmethanol, Trimethylsilylethanol, Dimethylphenylsilylmethanol, Hydroxymethyltriethoxysilan, 3-Aminopropyltrimethoxysilan oder 3-Aminopropyltriethoxysilan in Betracht. Weiterhin kommen als Bausteine Hydroxyacrylate in Betracht, wie zum Beispiel Hydroxyethylacrylat, Hydroxypropylacrylat, Hydroxybutylacrylat, Hydroxyethylmethacrylat, Hydroxyalkylacrylamide, wie zum Beispiel Hydroxymethylacrylamid oder Hydroxymethylmethacrylamid.

Wird ein erfindungsgemäßer Baustein nun zur Funktionalisierung einer Verbindung oder Oberfläche, die mit Isocyanat reaktive Gruppen aufweist, verwendet, so ist es zweckmäßig, aber nicht zwingend erforderlich, diesen Baustein in situ zu erzeugen und ihn anschließend mit der Verbindung oder Oberfläche, die mit Isocyanat reaktive Gruppen aufweist, umzusetzen. Dabei erfolgt die Auswahl des Isocyanats und des Molekülrestes -(Y)$_n$ der mit Isocyanat reaktiven Verbindung der allgemeinen Formel 3 gemäß den Eigenschaften, welche die Verbindung oder Oberfläche, die mit Isocyanat reaktive Gruppen aufweist, nach der Funktionalisierung oder Modifizierung mit den Bausteinen aufweisen soll.

Die Funktionalisierung oder Modifizierung einer Verbindung oder einer Oberfläche erfolgt, indem die erfindungsgemäßen NCO-Gruppen aufweisenden Bausteine rein oder in einem Lösungsmittel mit der zu funktionalisierenden oder zu modifizierenden Verbindung oder Oberfläche zusammengebracht werden. Die Reaktion zwischen den NCO-Gruppen der erfindungsgemäßen Bausteine und den mit NCO-Gruppen reaktiven Gruppen der zu funktionalisierenden oder zu modifiziernden Verbindung oder Oberfläche findet gegebenenfalls unter Zugabe von Katalysatoren vorzugsweise bei Temperaturen zwischen 0 und 120°C und bei Reaktionszeiten zwischen 5 Minuten und 24 Stunden statt. Dabei können die erfindungsgemäßen Bausteine auch unterstöchiometrisch bezogen auf die funktionellen Gruppen des zu funktionalisierenden oder zu modifiziernden Substrats eingesetzt werden, um gewünschtenfalls eine Teilmodifikation an der Verbindung oder an der Oberfläche durchzuführen.

In einer bevorzugten Ausführungsform der vorliegenden Erfindung ist die Verbindung, die mit Isocyanat reaktive Gruppen aufweist, ein Monomer oder ein Polymer, das funktionelle Gruppen, bevorzugt als Endgruppen oder als Seitengruppen, besitzt, die mit Isocyanat reaktiv sind. Beispiele für Monomere sind OH- oder NH-Gruppen aufweisende Substanzen, wie Ethylenglykol, Propylenglykol, Butandiol, Pentandiol, Hexandiol, Glycerin, Trimethylolpropan, Pentaerythrit, Sorbit, Zucker, Ethylendiamin, Butylendiamin, Hexylendiamin oder Melamin. Beispiele für Polymere sind OH-Gruppen-haltige Polymere, wie Polyetherpolyole, Polyesterpolyole, Polyacrylatpolyole, Polyvinylalkohole, Polybutadienalkohole, NH-Gruppen aufweisende Polymere, wie Aminogruppen-terminierte Polyetherole, Polyalkylenimine, Polyalkylenamine, Polyvinylimidazole, Polyamidoamine, Säure- oder Säureanhydrid-Gruppen aufweisende Polymere, wie Polyacrylsäuren oder Maleinsäureanhydrid-Gruppen enthaltende Polymere.

Bevorzugt sind dabei Polymere, die OH- oder NH-Gruppen aufweisen, wie Polyetherole, Polyesterole, Polyacrylatpolyole, Polyethylenimine oder Polyvinylamine.

In einer weiteren Ausführungsform der vorliegenden Erfindung ist die Verbindung, die mit Isocyanat reaktive Gruppen aufweist, ein Dendrimer, ein hoch- oder hyperverzweigtes Polymer oder ein Sternpolymer, jeweils mit, beispielsweise durch Hydroxy-, Amino-, Carboxy-, Thiol- oder NCO-reaktive Silangruppen, funktionalisierter Molekülperipherie.

Unter Dendrimeren werden im Rahmen dieser Erfindung Makromoleküle verstanden, die strukturell und molekular einheitlich sind und, ausgehend von einem Kernmolekül, verzweigte Molekülketten aufweisen. Ein hoch- oder hyperverzweigtes Polymer bedeutet im Rahmen dieser Erfindung ein Polymer, das durch intermolekulare Polymerisation von Molekülen des Typs $AB_n$ entsteht, wobei A und B miteinander reagieren können und n vorzugsweise eine Zahl von 1 bis 3 ist. Diese Moleküle weisen ähnlich wie Dendrimere einen hohen Verzweigungsgrad auf, sind jedoch strukturell uneinheitlich und durch eine Molmassenverteilung gekennzeichnet.

Ein Sternpolymer ist als Spezialfall einer dendrimeren oder hoch-oder hyperverzweigten Struktur aufzufassen. Hier werden ausgehend von einem einzelnen Kernmolekül oder einem Oligomer mit niedrigem Verzweigungsgrad als Kern polymere Ketten, die vorzugsweise linear oder gering verzweigt sind, sternförmig an den Kern angefügt.

Eine genauere Beschreibung der genannten Strukturen ist z.B. in E. Malmström und A. Hult, J.M.S.-Rev. Macromol. Chem. Phys., 1997, C 37(3), 555-579, "Dendritic Molecules", G.R. Newkome, C.N. Moorefield, F. Vögtle, Verlag Chemie, Weinheim 1996 und "Topics in Current Chemistry No. 197, Dendrimers", F. Vögtle, Springer-Verlag, Berlin-Heidelberg, 1998 und J. Huybrechts und K. Dusek, Surface Coatings International 1998, 3, 117-127, zu finden.

In einer weiteren Ausführungsform können die Bausteine der vorliegenden Erfindung dazu verwendet werden, verschiedene Oberflächen, die mit Isocyanat reaktive Gruppen aufweisen, zu funktionalisieren oder modifizieren. Dabei erfolgt die Wahl der Eigenschaften, welche die Oberfläche, die mit Isocyanat reaktive Gruppen aufweist, nach der Funktionalisierung oder Modifizierung mit den Bausteinen aufweisen soll, durch Auswahl des Isocyanats und des Molekülrestes -$(Y)_n$ der mit Isocyanat reaktiven Verbindung der allgemeinen Formel 3. Die in der vorliegenden Erfindung verwendeten Oberflächen können beispielsweise ausgewählt sein aus (in Klammern sind die jeweiligen, mit Isocyanat reaktiven Gruppen angegeben):

Glas (OH), Holz (OH), Textilien, zum Beispiel aus Baumwolle (OH) oder Wolle (OH, NH, SH, COOH), Leder (OH, NH, SH, COOH), Papier (OH), Kunststoff (OH, NH, SH, COOH), Keramik (OH), Mauerwerk (OH), Beton (OH) oder teilweise oxidierten Metallen (OH).

Bevorzugt verwendete Oberflächen sind Glas, Holz, Papier und Textilien.

Die Erfindung wird nachfolgend durch Beispiele näher erläutert.

Beispiele

1. Herstellung von NCO-Gruppen enthaltenden Monourethanen und Monoharnstoffen

1.1. Monourethane aus Hexamethylendiisocyanat (HDI)

1680 g HDI (10 mol) und 0,84 g Dibutylzinndilaurat (500 ppm bez. HDI) wurden unter Stickstoffbedeckung vorgelegt und auf 60°C erwärmt. Bei dieser Temperatur wurden innerhalb 30 min 2 mol der jeweilige Alkohole nach Tabelle 1 zugetropft. Man ließ 30 min bei 60°C nachreagieren. Anschließend wurde das Produkt durch Destillation am Dünnschichtverdampfer im Vakuum vom monomeren HDI befreit. Die Daten zu den Produkten 1 bis 3 stehen in Tabelle 1.

1.2. Monourethan aus 2,4-Toluylendiisocyanat (TDI)

Das aromatische Diisocyanat wurde unter Stickstoffbedeckung in trockenem Chlorbenzol gelöst, auf 80°C aufgeheizt und die OH-Komponente nach Tabelle 1 bei dieser Temperatur innerhalb 30 min zugetropft. Anschließend ließ man 60 min bei 80°C nachreagieren. Das Molverhältnis zwischen Isocyanat und dem jeweiligen Alkohol betrug hier 1:1. Als Nebenprodukt trat neben nicht umgesetztem Diisocyanat das Diaddukt auf, das ggf. durch Reinigungsoperationen (Chromatographie, Kristallisation) entfernt werden konnte. Die Daten zu dem Produkt 4 stehen in Tabelle 1.

Tabelle 1:

| OH-Komponenten als Reaktionspartner | | | | |
|---|---|---|---|---|
| Produkt Nr. | Isocyanat | Alkohol | NCO-Gehalt (Gew.%) | Kennzahlen |
| 1 | HDI | Octadecanol | 9,6 | Schmelzpunkt: 57 bis 58 °C |
| 2 | HDI | Cis-9-octadecen-1-ol | 9,6 | Schmelzpunkt: 28 bis 31 °C |
| 3 | HDI | Hydroxyethylmethacrylat | 14,1 | Viskosität bei 23°C, 80%ig in Ethylacetat: 77 mPas |
| 4 | TDI | Hydroxy-ethylacrylat | 14,5 | Schmelzpunkt: 38 bis 40°C |
| HDI = 1,6-Hexamethylendiisocyanat TDI = 2,4-Toluylendiisocyanat | | | | |

1.3. Monothiourethan aus Isophorondiisocyanat (IPDI)

222 g IPDI (1 mol) wurden unter Stickstoffbedeckung vorgelegt und auf 50°C erwärmt. Bei dieser Temperatur wurden innerhalb 30 min 1 mol Mercaptoessigsäure, gelöst in 200 ml trockenem Chlorbenzol, zugetropft. Man ließ 60 min bei 50°C nachreagieren. Das Additionsprodukt wurde vorzugsweise nicht aufgereinigt oder isoliert, sondern direkt mit den zu modifizierenden oder zu funktionalisierenden Molekülen oder Oberflächen weiter umgesetzt. Die Daten zu dem Produkt 5 stehen in Tabelle 2.

1.4.Monoharnstoffe aus Diisocyanaten und Aminen oder Alkanolaminen

1 Mol des jeweiligen Isocyanats wurde in 300 ml THF (trocken) gelöst und auf 10°C gekühlt. Anschließend wurde innerhalb 30 min 1 Mol entsprechendes Amin, gelöst in 100 ml THF, zugegeben, wobei die Temperatur bei 10°C gehalten wurde. Es wurde noch 30 min bei 10°C nachgerührt. Die Produkte aus TMXDI erwiesen sich als überraschend stabil und waren über längere Zeit (ca. 24 h) bei Raumtemperatur ohne Polymerisation haltbar. Die Addukte aus IPDI wurden vorzugsweise nicht isoliert, sondern direkt mit den zu modifizierenden oder zu funktionalisierenden Molekülen oder Oberflächen weiter umgesetzt. Daten zu den erfindungsgemäßen Produkten 6 bis 11 stehen in Tabelle 2.

Tabelle 2:

| SH und COOH-Gruppen sowie NH- und OH-Gruppen enthaltende Komponenten als Reaktionspartner | | | | |
|---|---|---|---|---|
| Produkt Nr. | Isocyanat | Reaktivkomponente | NCO-Gehalt (Gew.-%) | Reinheit (GPC F1.-%) |
| 5 | IPDI | Mercaptoessigsäure | 13,4 | 90,0 |
| 6 | IPDI | Dodecylamin | 10,3 | 99,0 |
| 7 | IPDI | Isopropanolamin | 14,1 | 91,0 |
| 8 | IPDI | Diisopropanolamin | 11,8 | 99,0 |
| 9 | TMXDI | Diethanolamin | 12,0 | 89,0 |
| 10 | TMXDI | Diisopropanolamin | 11,1 | 96,0 |
| 11 | TMXDI | Tris(hydroxy-methyl)-aminomethan | 11,5 | 91,0 |
| IPDI = Isophorondiisocyanat TMXDI = Tetramethylxylylendiisocyanat | | | | |

2. Modifizierung von Makromolekülen

2.1. Modifizierung eines Polyamin-Dendrimeren der $NH_2$-Funktionalität 8 gemäß WO 93/14147, kommerziell erhältlich als ASTRA-MOL®-Typ von der Firma DSM N.V..

Das Reaktionsprodukt aus HDI und Octadecenol (Monourethan 2 aus Tabelle 1) wurde in trockenem THF vorgelegt, auf 10°C abgekühlt und bei dieser Temperatur das dendrimere Polyamin (M = 773 g/mol), gelöst in THF, innerhalb 30 min zugegeben. Anschließend wurde 1 h bei 23°C nachgerührt. Die Zugabemenge wurde so berechnet, daß pro Mol der NCO-Gruppen ein Mol der $NH_2$-Gruppen des Polyamins zur Reaktion kam. Das ausgefallene Produkt wurde abfiltriert, mit THF gewaschen und im Vakuum bei 40°C getrocknet. Ausbeute 93 % der Theorie, Schmp. 119 bis 120°C.

Während sich das Polyamin-Dendrimer z.B. in Wasser oder Ethanol löste, war das modifizierte Produkt darin nicht löslich. Das modifizierte Dendrimer löste sich dagegen z.B. in Chlorbenzol, n-Heptan oder Isooctan.

2.2. Modifizierung eines Polyethylenimins

118 g des Reaktionsproduktes aus HDI und Octadecenol (Monourethan 2 aus Tabelle 1) wurden in 400 ml trockenem THF vorgelegt und bei Raumtemperatur (23°C) wurden 10,5 g Polyethylenimin (Mn = 700 g/mol), gelöst in 100 ml destilliertem Wasser, innerhalb 30 min zugegeben. Anschließend wurde 4 h bei 23°C nachgerührt. Das Reaktionsgemisch wurde mit 2 1 Aceton versetzt, gut durchgerührt und 12 h stehengelassen. Der ausgefallene Feststoff wurde abfiltriert, mit Aceton gewaschen und im Vakuum bei 40°C getrocknet.

Die Ausbeute betrug 89 % der Theorie, der Schmelzpunkt lag bei 119 bis 121°C.

Während sich das Polyethylenimin in Wasser löste, war das modifizierte Produkt darin unlöslich. Es löste sich dagegen z.B. in Chlorbenzol oder Butylacetat.

2.3. Funktionalisierung eines hochfunktionellen Polyacrylat-alkohols

(Lumitol® H 136, BASF AG)

25 g des Adduktes aus TDI und Hydroxyethylacrylat (Monourethan 4 aus Tabelle 1) wurden in 100 ml trockenem Chlorbenzol gelöst und auf 40°C erwärmt. Dazu wurde innerhalb 1 h die Lösung aus 68,7 g Lumitol® H 136, gelöst in 100 ml Butylacetat, getropft und anschließend 4 h bei 40°C gerührt. Nach dieser Zeit war mittels IR-Spektroskopie im Produktgemisch keine NCO-Bande mehr festzustellen. Nach Abziehen des Lösungsmittels wurde ein Acrylatgruppen aufweisender Feststoff mit einem Schmelzbereich von 31 bis 35°C erhalten.

3. Modifizierung von Oberflächen: Hydrophobierung eines Baumwoll-Gewebes

Es wurden parallel zwei Lösungen (Lösung 1 und Lösung 2), bestehend aus 100 ml trockenem Tetrahydrofuran und 50 mg Dibutylzinndilaurat, vorbereitet. In Lösung 1 wurden zusätzlich 2 g des Adduktes aus HDI und Octadecenol (Monourethan 2 aus Tabelle 1) gegeben und gelöst. Anschließend wurde zu beiden Lösungen je ein 5 x 10 cm großes Stück eines Baumwollgewebes gegeben und die Reaktionslösungen mit den Gewebestücken parallel 2 h auf 60°C erwärmt. Nach dem Abkühlen wurden die Baumwollgewebe aus den Lösungen genommen, mit jeweils 2 x 100 ml Tetrahydrofuran gewaschen und mit einem Heißluft-Gebläse vollständig getrocknet. Anschließend wurden die Gewebe mit Wasser benetzt. Während das unbehandelte Gewebe aus Lösung 2 sich mit Wasser vollsaugte, perlte das Wasser von dem mit Monourethan modifizierten Gewebe vollständig ab.

**Patentansprüche**

**1.** Verbindung der allgemeinen Formel 1,

$$\text{OCN-R}^1\text{-NHCOX-R}^2\text{-(Y)}_n \tag{1},$$

in der $R^1$ und $R^2$ ein substituierter oder unsubstituierter, linearer oder verzweigter, gesättigter oder ungesättigter Alkylenrest mit 1 bis 20 C-Atomen, ein substituierter oder unsubstituierter, gesättigter oder ungesättigter Cycloalkylenrest mit 3 bis 20 C-Atomen, ein substituierter oder unsubstituierter Arylenrest mit 3 bis 20 C-Atomen, ein Arylenalkylenrest mit 4 bis 20 C-Atomen, ein heterocyclischer Rest oder eine beliebige lineare oder verzweigte Abfolge von zwei oder mehr der genannten Reste, gegebenenfalls verknüpft über Ether-, Thioether-, Ester-, Amin- oder Amid-Strukturen, ist, X eine kovalente Bindung zu $R^2$ ist oder O, S oder $NR^3$ bedeutet, wobei $R^3$ ein Wasserstoffatom oder ein substituierter oder unsubstituierter, linearer oder verzweigter, gesättigter oder ungesättigter Alkylrest mit 1 bis 20 C-Atomen, ein substituierter oder unsubstituierter, gesättigter oder ungesättigter Cycloalkylrest mit 3 bis 20 C-Atomen, ein substituierter oder unsubstituierter Arylrest mit 3 bis 20 C-Atomen, ein heterocyclischer Rest oder eine beliebige lineare oder verzweigte Abfolge von zwei oder mehr der genannten Reste ist, Y ein Wasserstoffatom oder eine freie funktionelle Gruppe bedeutet und n für eine ganze Zahl von 1 bis 20 steht.

2. Verbindung nach Anspruch 1, wobei Y eine freie funktionelle Gruppe, die aus der Gruppe der Hydroxyl-, Amino-, Amido-, Carbonyl-, Carboxyl-, Mercapto-, Sulfonyl-, Sulfinyl-, Sulfenyl-, Sulfat-, Nitro-, Nitril-, Isonitril-, Cyanat-, Silyl-, Silanyl-, Phosphin-, Phosphat-, Phosphit-, Phosphonat-, Acrylat-, Methacrylat-, Allyl- oder Vinylgruppen oder deren Gemischen ausgewählt ist, bedeutet.

3. Verfahren zur Herstellung einer Verbindung der allgemeinen Formel 1

$$OCN\text{-}R^1\text{-}NHCOX\text{-}R^2\text{-}(Y)_n \tag{1}$$

durch Umsetzung eines Diisocyanats der allgemeinen Formel 2

$$OCN\text{-}R^1\text{-}NCO \tag{2}$$

mit einer Verbindung der allgemeinen Formel 3

$$HX\text{-}R^2\text{-}(Y)_n \tag{3},$$

wobei $R^1$, $R^2$, $R^3$, X, Y und n gemäß Anspruch 1 oder 2 definiert sind und X in Formel 3 außerdem OCO bedeuten kann.

4. Verfahren zur Funktionalisierung oder Modifizierung von Verbindungen oder Oberflächen, die mindestens eine mit Isocyanat reaktive Gruppe aufweisen, durch Umsetzung einer Verbindung der allgemeinen Formel 1

$$OCN\text{-}R^1\text{-}NHCOX\text{-}R^2\text{-}(Y)_n \tag{1},$$

in der $R^1$ und $R^2$ ein substituierter oder unsubstituierter, linearer oder verzweigter, gesättigter oder ungesättigter Alkylenrest mit 1 bis 20 C-Atomen, ein substituierter oder unsubstituierter, gesättigter oder ungesättigter Cycloalkylenrest mit 3 bis 20 C-Atomen, ein substituierter oder unsubstituierter Arylenrest mit 3 bis 20 C-Atomen, ein Arylenalkylenrest mit 4 bis 20 C-Atomen, ein heterocyclischer Rest oder eine beliebige lineare oder verzweigte Abfolge von zwei oder mehr der genannten Reste, gegebenenfalls verknüpft über Ether-, Thioether-, Ester-, Amin- oder Amid-Strukturen, ist, X eine kovalente Bindung zu $R^2$ ist oder O, S oder $NR^3$ bedeutet, wobei $R^3$ ein Wasserstoffatom oder ein substituierter oder unsubstituierter, linearer oder verzweigter, gesättigter oder ungesättigter Alkylrest mit 1 bis 20 C-Atomen, ein substituierter oder unsubstituierter, gesättigter oder ungesättigter Cycloalkylrest mit 3 bis 20 C-Atomen, ein substituierter oder unsubstituierter Arylrest mit 3 bis 20 C-Atomen, ein heterocyclischer Rest oder eine beliebige lineare oder verzweigte Abfolge von zwei oder mehr der genannten Reste ist, Y ein Wasserstoffatom oder eine freie funktionelle Gruppe bedeutet und n für eine ganze Zahl von 1 bis 20 steht, mit mindestens einer mit Isocyanat reaktiven Gruppe einer Verbindung, die mindestens eine mit Isocyanat reaktive Gruppe aufweist, oder mit mindestens einer mit Isocyanat reaktiven Gruppe auf einer Oberfläche, die mindestens eine mit Isocyanat reaktive Gruppe aufweist.

5. Verfahren nach Anspruch 4, wobei Y eine freie funktionelle Gruppe, die aus der Gruppe der Hydroxyl-, Amino-, Amido-, Carbonyl-, Carboxyl-, Mercapto-, Sulfonyl-, Sulfinyl-, Sulfenyl-, Sulfat-, Nitro-, Nitril-, Isonitril-, Cyanat-, Silyl-, Silanyl-, Phosphin-, Phosphat-, Phosphit-, Phosphonat-, Acrylat-, Methacrylat-, Allyl- oder Vinylgruppen oder deren Gemischen ausgewählt ist.

6. Verfahren nach Anspruch 4 oder 5, wobei die Verbindung, die mindestens eine mit Isocyanat reaktive Gruppe aufweist, Monomere, Polymere, Dendrimere, hyperverzweigte Polymere oder Sternpolymere, die mindestens eine mit Isocyanat reaktive Gruppe aufweisen, umfaßt.

7. Verfahren nach Anspruch 6, wobei Polymere, die mindestens eine mit Isocyanat reaktive Gruppe aufweisen, aus der Gruppe der Polyetherpolyole, Polyesterpolyole, Polyacrylatpolyole, Polyvinylalkohole, Polyalkylenimine, Polyalkylenamine, Polyamidoamine, Polyacrylsäuren oder Säureanhydridgruppen tragenden Polymeren ausgewählt sind.

8. Verfahren nach Anspruch 4 oder 5, wobei die Oberfläche, die mindestens eine mit Isocyanat reaktive Gruppe aufweist, eine Oberfläche aus Holz, Glas, Textilien, keramischen Materialien, Leder, Papier, Kunststoff, Stein, Beton, Metallen oder Metallegierungen umfaßt, mit der Maßgabe, daß diese Oberflächen mindestens eine mit Isocyanat reaktive Gruppe aufweisen.

9. Verfahren nach einem der Ansprüche 4 bis 8, wobei die mit Isocyanat reaktive Gruppe aus der Gruppe der Hydroxyl-, Amino-, Amido-, Carboxyl- oder Mercaptogruppe oder deren Gemischen ausgewählt ist.

10. Verwendung einer Verbindung der allgemeinen Formel 1

$$OCN-R^1-NHCOX-R^2-(Y)_n \qquad (1),$$

in der $R^1$, $R^2$, X, Y und n gemäß Anspruch 4 oder 5 definiert sind, zur Funktionalisierung oder Modifizierung von Verbindungen oder Oberflächen, die mindestens eine mit Isocyanat reaktive Gruppe aufweisen.